# EUROPEAN PATENT SPECIFICATION

(11) **EP 4 591 805 B1**
(45) Date of publication and mention of the grant of the patent: **18.11.2026**
(21) Application number: 25180812.7
(22) Date of filing: 30.09.2019
(51) Int. Cl.: A61B 17/04

(54) **SYSTEM AND METHOD FOR REPAIRING SOFT TISSUE TEARS**
SYSTEM UND VERFAHREN ZUR REPARATUR VON WEICHTEILRISSEN
SYSTÈME ET PROCÉDÉ DE RÉPARATION DE DÉCHIRURES DE TISSU MOU

(30) Priority: 28.09.2018 US 201862738551 P; 20.12.2018 US 201862782689 P; 01.03.2019 US 201962791127 P
(43) Date of publication of application: 30.07.2025
(62) Document number(s) of the earlier application(s) in accordance with Art. 76 EPC: 19790951.8 / 3 856 044
(73) Proprietor: Conmed Corporation, Largo, FL 33773 (US)
(72) Inventor: LOMBARDI, Giuseppe, New Port Richey, FL 34655 (US)
(74) Representative: Strehl & Partner mbB

(56) References cited:
- EP-A1- 3 248 549
- US-A- 6 066 146
- US-A1- 2002 107 532
- US-A1- 2016 113 643

## Description

### BACKGROUND OF THE INVENTION

### 1. Field of the Invention

The present invention is directed generally to surgical tools and instruments and, more particularly, to a system for repairing soft tissue tears such as meniscal tears.

### 2. Description of Related Art

The meniscus is a piece of cartilage located within the knee joint, between the top of the tibia and the bottom of the femur. The meniscus serves to facilitate stable movement of the tibia and femur relative to one another, and to absorb shock and to spread load. The meniscus is frequently damaged (e.g., torn) as the result of injury and/or accident. A damaged meniscus can impede proper motion of the knee joint and cause pain, among other problems.

More particularly, the essential role of an intact meniscus, and its importance for proper knee function, has been well documented and accepted by the general orthopedic community. An intact and functioning meniscus is critical to optimally distribute weightbearing forces that transfer through the knee joint while maintaining knee stability. The meniscus is also vital to preserving the articular cartilage surfaces of the knee. Loss of meniscal tissue is considered to be a key precursor to the development of knee osteoarthritis.

A major challenge in repairing a torn meniscus is the fact that the tissue itself is a fibrous structure that is not uniformly vascular. The vascular zones of the meniscus comprise about one third of the meniscus tissue and are generally recognized as the "red-red" and "red-white" zones. The "red-red" zone (i.e., the most highly vascularized portion of the meniscus) is an area in which meniscal repairs are known to heal easily and is located along its outer periphery. The "red-white" zone extends from the most vascular area towards the inner portions of the meniscus where the blood supply eventually declines to nonvascular tissue (which is sometimes referred to as the "white-white" zone). It is believed that proper surgical technique is of great importance if a successful repair is to be achieved in the "red-white" zone. It is generally accepted knowledge that about 15% of all meniscal tears occur in the "red-red" zone, another 15% of meniscal tears occur in the "red-white" zone, and the remaining 70% of meniscal tears occur in the "white-white" (or non-vascularized) zone of the meniscus.

Another significant challenge in repairing a torn meniscus is that the size and shape of the tears vary, making the reduction and apposition of the torn tissue difficult to accomplish. Without proper apposition and stability, torn meniscal tissue will not heal properly.

The art of repairing torn meniscal tissue was first developed and pioneered throughout the 1980s by early sports medicine-focused surgeons. The earliest methods employed only suture in the repair. The techniques of "inside-out" and "outside-in" suturing became the so-called "gold standard for the repair of meniscal tissue. Both of these techniques focused on passing small diameter suture (size 2-0 or 3-0) through the meniscus, reducing and closing the tear, and then tying a suture knot over the knee capsule so as to fixate and stabilize the tear. A feature of these early all-suture repairs was that the surface of the meniscus was kept relatively smooth since the suture knot was outside of the knee joint, and the use of a needle and suture allowed the surgeon a great deal of flexibility in adequately reducing and stabilizing the tear.

Eventually, these early surgeons began concomitant use of complementary techniques to promote a vascular response in the more non-vascular areas of the meniscus. Methods such as tear edge and meniscapsular rasping, the application of an interpositional blood clot, trephination to create a vascular channel, and fascial sheath or synovial flap coverage have been shown in several studies to be 150% more effective in healing a torn meniscus when compared to repairs that do not use such concomitant techniques.

The specific issues and challenges associated with the aforementioned all-suture inside-out and outside-in repair techniques are centered primarily on issues relating to the "user interface" and to the "tethering" of the meniscus to the knee capsule. More particularly, the "user interface" issues generally relate to the technical demands required in the operating room: the skill of the surgeon and the number of assistants required to safely pass the needle and suture from the anterior portion of the meniscus through the posterior portion of the meniscus and exit out through the posterior/medial aspect of the knee joint (i.e., the so-called "inside-out" technique); or the passing of a needle and suture from the medial aspect of the exterior of the knee into the knee joint, through the meniscus, the retrieval and re-insertion back into the meniscus, and then passage back out through the capsule to the medial aspect of the knee (i.e., the so-called "outside-in" technique). The aforementioned tethering issues relate to more recent concerns about fixating suture over the knee capsule and thereby "tethering" the meniscus to the knee capsule, since evidence suggests that such tethering of the meniscus to the knee capsule may interfere with the normal biomechanics of the meniscus (e.g., load and force distribution, etc.).

As recognition of the importance of the meniscus grew in the late 1980s, new methods of meniscus repair were developed. These new methods focused on improving execution of the procedure in order to make it easier, simpler and faster to accomplish. The new gold standard approach became the so-called "all-inside" technique. The all-inside technique is intended to not violate the knee capsule or require any incisions on the posterior/medial aspects of the knee (i.e., such as is required with the inside-out and outside-in suturing techniques discussed above). With the all-inside technique, the entire repair both approximation and fixation is performed intra-articularly.

The first all-inside repair devices were tack-like implants that were inserted through a standard arthroscopic portal and then forcefully pushed through the meniscus, crossing through the tear, thereby closing and fixing the tear without the use of suture. These tack-like implants were formed out of biomaterials such as PLA, PLLA or PGA that were expected to biodegrade over time. However, these materials are quite hard when first inserted and, in use, were found to degrade or bioabsorb much more slowly than anticipated. Clinical use and follow-up have demonstrated the inherent risks associated with the use of tack-like implants within the knee joint, as numerous published studies have reported device failure which can lead to tear reformation, loose implants within the knee joint and articular cartilage damage. Furthermore, it can be challenging for the surgeon to adequately address various tear shapes and sizes using these tack-like implants.

As a result, attention has returned to suture-based repairs, with a new focus on performing a suture-based repair using an all-inside technique. There are several recent systems that seek to accomplish this goal. However, none of these systems have been found to be completely satisfactory.

Thus, there is a need for a new and improved apparatus for meniscal repair.

An implant attachment system having a button actuator for moving a pusher in a needle between different positions is disclosed in EP 3 248 549 A1. Further implant attachment devices related to the one of the present invention are disclosed in US 6 066 146 A, US 2002/107 532 A1 and US 2016/113 643 A1.

### SUMMARY OF THE INVENTION

Embodiments are directed to a system and method, which is not part of the invention, for repairing soft tissue tears such as meniscal tears. According to one aspect, the anchor system has a first implant connected to a length of suture. The length of suture is folded such that a tensioning limb extends from the first second implant fixed to the locking limb and an adjustment mechanism in the length of suture between the first implant and the second implant. The tensioning limb is passed through the adjustment mechanism.

The present invention is defined in Claim 1.

Further advantageous features are set out in the dependent claims.

According to an aspect, the present invention is a delivery device. The delivery device incudes an elongated body with a needle extending distally therefrom and a pusher assembly within the elongated body. The pusher assembly has a cannulated pusher rod extending distally from a pusher body and an actuator extending from the pusher body through the elongated body. The cannulated pusher rod is slidable within the needle. The delivery device also includes a locking mechanism on the elongated body which is movable between a locked position and unlocked position. The actuator is moveable from a first configuration to a second configuration when the locking mechanism is in the locked position and the actuator is moveable from the second configuration to a third configuration when the locking mechanism is in the unlocked position.

Another aspect concerns a method for meniscal repair, which is not part of the invention. The method includes the steps of: (i) providing a delivery device an elongated body with a needle extending distally therefrom, a pusher assembly within the elongated body, the pusher assembly comprising a cannulated pusher rod extending distally from a pusher body and an actuator extending from the pusher body through the elongated body, wherein the cannulated pusher rod is slidable within the needle, and a locking mechanism on the elongated body, the locking mechanism movable between a locked position and unlocked position; (ii) providing an anchor system having a first implant connected to a length of suture, the length of suture folded such that a tensioning limb extends from the first implant and a locking limb extends from the first implant, a second implant fixed to the locking limb, and an adjustment mechanism in the length of suture between the first implant and the second implant, wherein the tensioning limb is passed through the adjustment mechanism; (iii) positioning the needle at a first piercing location on a first side of a tissue; (iv) piercing the needle through the first piercing location to a second side of the tissue; (v) moving the actuator distally, deploying the first implant from the delivery device; (vi) removing the needle from the first piercing location on the first side of the tissue; (vii) positioning the needle at a second piercing location on a second side of the tissue; (viii) piercing the needle through the first piercing location to a second side of the tissue; (ix) moving the locking mechanism from the locked position to the unlocked position; and (x) moving the actuator farther distally, deploying the second implant from the delivery device.

These and other aspects of the invention will be apparent from and elucidated with reference to the embodiment(s) described hereinafter.

### BRIEF DESCRIPTION OF THE DRAWINGS

One or more aspects of the present invention are particularly pointed out and distinctly claimed as examples in the claims at the conclusion of the specification. The foregoing and other objects, features, and advantages of the invention are apparent from the following description taken in conjunction with the accompanying drawings in which:
FIG. 1 is a side perspective view schematic representation of an anchor system, according to an embodiment;
FIG. 2 is a perspective view schematic representation of the implant of the anchor system, according to an embodiment;
FIG. 3A is a perspective view schematic representation of the first step in creating a pierce hitch in the suture, according to an embodiment;
FIG. 3B is a perspective view schematic representation of the second step in creating a pierce hitch in the suture, according to an embodiment;
FIG. 3C is a perspective view schematic representation of the third step in creating a pierce hitch in the suture, according to an embodiment;
FIG. 3D is a perspective view schematic representation of the final step in creating a pierce hitch in the suture, according to an embodiment;
FIG. 4 is a perspective view schematic representation of the length of suture connected to the second implant, according to an embodiment;
FIG. 5 is a perspective view schematic representation of the anchor system in a pre-deployment configuration, according to an embodiment;
FIG. 6 is a perspective view schematic representation of the anchor system in the pre-deployment configuration, according to an embodiment;
FIG. 7 is a side view schematic representation of an anchor system, according to an alternative embodiment;
FIG. 8 is a perspective view schematic representation of the anchor system of FIG. 7;
FIG. 9 is another perspective view schematic representation of the anchor system of FIG. 7;
FIG. 10 is a perspective view schematic representation of an anchor system, according to another alternative embodiment;
FIG. 11 is a perspective view schematic representation of a delivery device, according to an embodiment;
FIG. 12 is a perspective view schematic representation of a pusher assembly of the delivery device, according to an embodiment;
FIG. 13 is a side view schematic representation of the delivery device in a first configuration, according to an embodiment;
FIG. 14 is a side view schematic representation of the delivery device in a second configuration, according to an embodiment;
FIG. 15 is a side view schematic representation of the delivery device with the locking mechanism in the unlocked position, according to an embodiment;
FIG. 16 is a side view schematic representation of the delivery device in a third configuration, according to an embodiment;
FIG. 17 is a side view schematic representation of the delivery device in a fourth configuration, according to an embodiment;
FIG. 18 is a top view schematic representation of a delivery device, according to an alternative embodiment;
FIG. 19 is a side view schematic representation of the delivery device, according to an alternative embodiment;
FIG. 20 is a sectional side view schematic representation of the delivery device, according to an alternative embodiment;
FIG. 21 is a sectional side view schematic representation of the delivery device in a first configuration, according to an alternative embodiment;
FIG. 22 is a sectional side view schematic representation of the delivery device in a second configuration, according to an alternative embodiment;
FIG. 23 is a top view schematic representation of the anchor system in a deployed configuration, according to an embodiment;
FIG. 24 is a top perspective view schematic representation of the anchor system in a deployed configuration, according to another embodiment;
FIG. 25 is a top perspective view schematic representation of the anchor system in a deployed configuration, according to an alternative embodiment;
FIG. 26 is a side perspective view schematic representation of the anchor system in the deployed configuration, according to an embodiment;
FIG. 27 is a top perspective view schematic representation of the anchor system in the deployed configuration, according to an embodiment;
FIG. 28 is a top perspective view schematic representation of the anchor system in the deployed configuration, according to an embodiment;
FIG. 29 is a top perspective view schematic representation of the delivery device deploying the anchor system, according to an embodiment;
FIG. 30 is a top perspective view schematic representation of the delivery device deploying the anchor system, according to an embodiment;
FIG. 31 is a top perspective view schematic representation of the delivery device deploying the anchor system, according to an embodiment;
FIG. 32 is a top perspective view schematic representation of the delivery device deploying the anchor system, according to an embodiment;
FIG. 33 is a top perspective view schematic representation of the delivery device deploying the anchor system, according to an embodiment;
FIG. 34 is a top perspective view schematic representation of the delivery device deploying the anchor system, according to an embodiment;
FIG. 35 is a top perspective view schematic representation of the delivery device deploying the anchor system, according to an embodiment;
FIG. 36 is a top perspective view schematic representation of the anchor system in the deployed configuration, according to an embodiment;
FIG. 37 is a top perspective view schematic representation of the anchor system in the deployed configuration, according to an embodiment; and
FIG. 38 is a top perspective view schematic representation of the anchor system in the deployed configuration, according to an embodiment.

### DETAILED DESCRIPTION OF THE INVENTION

Aspects of the present invention and certain features, advantages, and details thereof, are explained more fully below with reference to the non-limiting examples illustrated in the accompanying drawings. Descriptions of well-known structures are omitted so as not to unnecessarily obscure the invention in detail. It should be understood, however, that the detailed description and the specific non-limiting examples, while indicating aspects of the invention, are given by way of illustration only, and are not by way of limitation. Various substitutions, modifications, additions, and/or arrangements, within the scope of the underlying inventive concepts will be apparent to those skilled in the art from this disclosure.

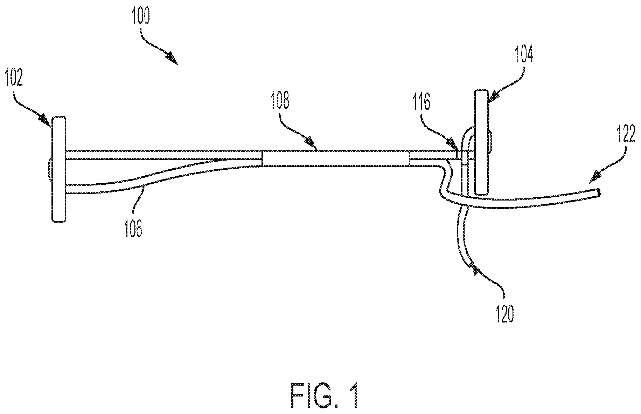

Referring now to the figures, wherein like reference numerals refer to like parts throughout, FIG. 1 shows a side perspective view schematic representation of an anchor system 100, according to an embodiment. The anchor system 100 includes a first implant 102 and a second implant 104 interconnected by a length of suture 106. The length of suture 106 terminates in a first end 120 and a second end 122. The length of suture 106 is used to adjust the position of the first implant 102 relative to the second implant 104 (and/or vice versa) by creating an one-way adjustable loop via an adjustment mechanism 108 and a locking mechanism 116. In some embodiments, the adjustment mechanism 108 is an eye splice (i.e., finger trap), while in other embodiments, the adjustment mechanism 108 is a sliding knot. The adjustment mechanism 108 can be any one-way adjustable locking construct. In an embodiment, the locking mechanism 116 is a pierce hitch. However, the locking mechanism 116 can be any fixed locking construct, such as a knot.

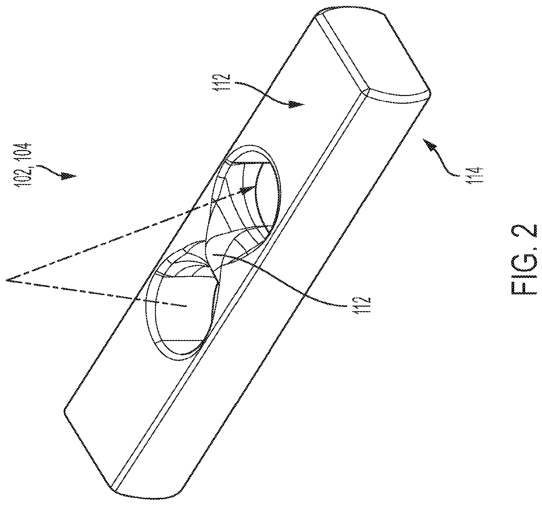

As shown in FIG. 2, the first and second implants 102, 104 are rectangular so they lay flat against the damaged tissue. However, the implants 102, 104 may have any other geometric configuration. The first and second implants 102, 104 can be composed of suture material, plastic, or any other suitable surgical material. As shown in FIG. 2, each implant 102, 104 has a pair of adjacent, spaced apertures 110. In the depicted embodiment, the pair of adjacent, spaced apertures 110 extend through a first side 112 of the implant 102, 104 to a second side 114 of the implant 102, 104. The implant 102, 104 has a radiused saddle 112 between the apertures 110 to maintain the suture 106 thereacross when the anchor system 100 is in a deployed configuration.

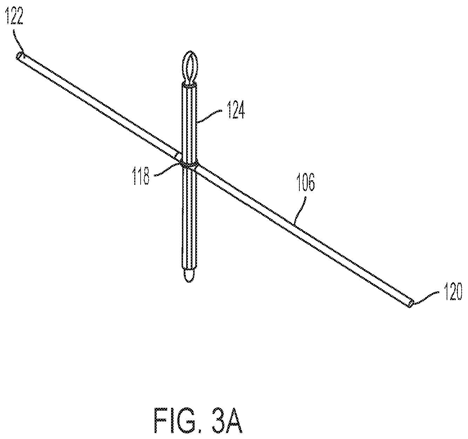

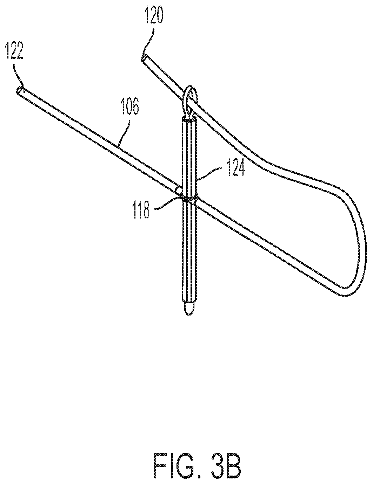

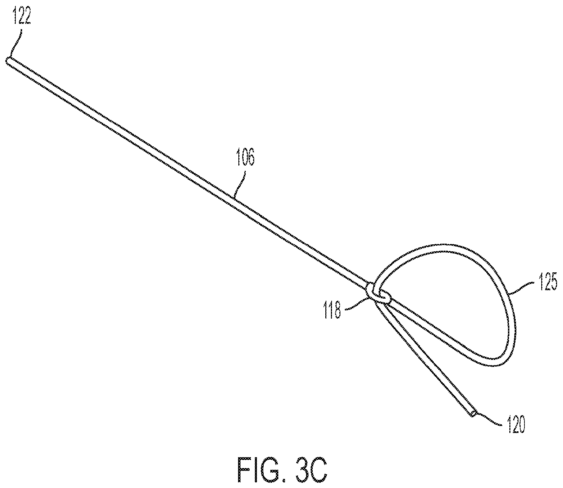

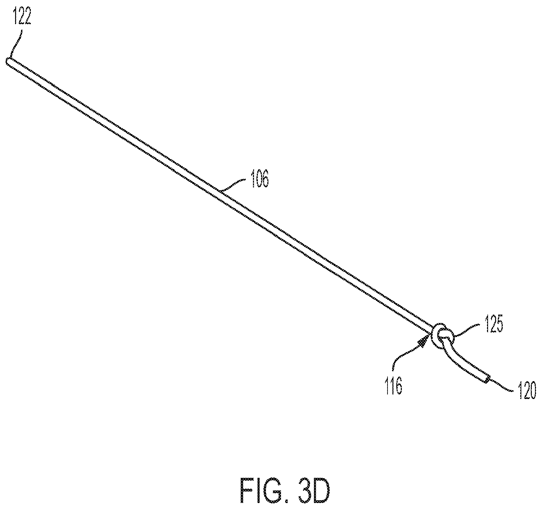

Turning now to FIGs. 3A-3D, there are shown perspective views schematic representations of a method, which is not part of the invention, for creating the locking mechanism 116 in the length of suture 106, according to an embodiment. In the embodiment shown in FIGs. 3A-3D, the locking mechanism 116 is a pierce hitch. To create a pierce hitch 116, a hole 118 is formed in the length of suture 106 between the first end 120 and the second end 122. A threader 124 or another similar device is placed through the hole 118 in the length of suture 106, as shown in FIG. 3A. Thereafter, the first end 120 of the suture 106 is threaded or woven through the threader 124, as shown in FIG. 3B. Then, the threader 124 is pulled through the hole 118 in the length of suture 106, creating a loop 125 in the suture 106, as can be seen in FIG. 3C. To minimize the loop 125, thereby creating the pierce hitch 116 shown in FIG. 3D, the first end 120 of the suture 106 is tensioned.

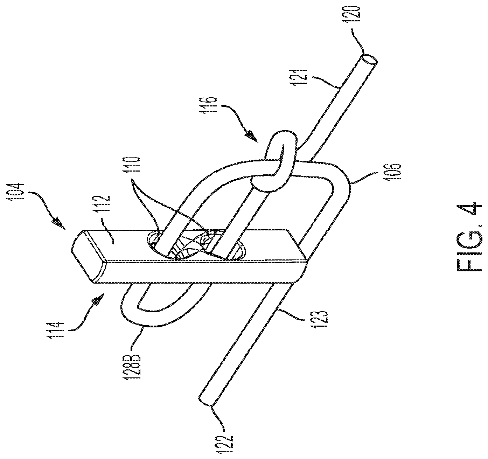

Referring now to FIG. 4, there is shown a perspective view schematic representation of the length of suture 106 connected to the second implant 104, according to an embodiment. In the depicted embodiment, the second implant 104 comprises the pair of adjacent, spaced apertures 110. The second end 122 of the length of suture 106 is first passed through one of the adjacent, spaced apertures 110 from a first side 112 of the implant 104. Then, the second end 122 of the length of suture 106 is passed through the other of the adjacent, spaced apertures 110 from a second side 114 of the implant 104 such that an intermediate portion 128B of suture 106 extends between the adjacent, spaced apertures 110 on the second side 114 of the implant 104 (over the radiused saddle 112). Finally, the second end 122 of the length of suture 106 is passed through the pierce hitch 116, as shown in FIG. 4, resulting in a "pierce hitch tail" 121 extending to the first end 120 of the suture 106 and a "tensioning limb" 123 extending to the second end 122 of the suture 106. The pierce hitch 116 functions as a self-collapsing noose around the pierce hitch tail 121 and is stationary relative to the position of the adjustment mechanism 108 (FIG. 1).

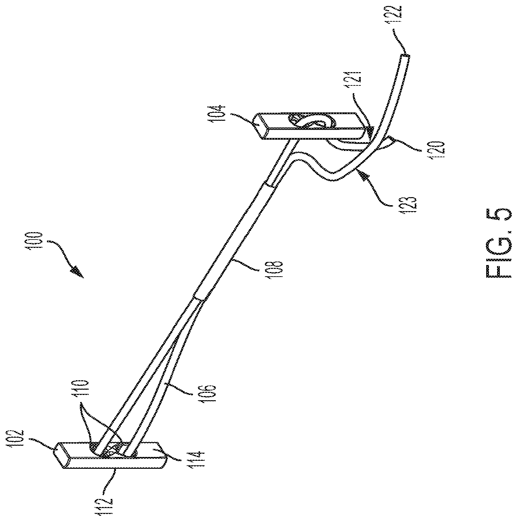

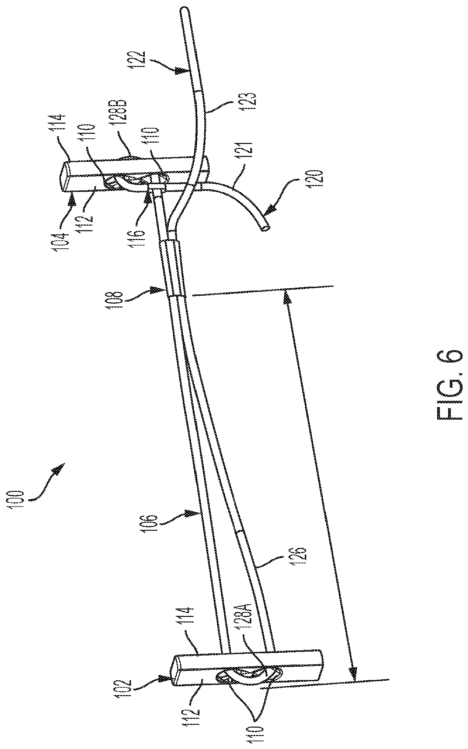

Turning now to FIG. 5, there is shown a perspective view schematic representation of the anchor system 100, according to an embodiment. From the configuration shown in FIG. 4, the first implant 102 is connected to the length of suture 106. The second end 122 of the length of suture 106 is passed through one of the pair of adjacent, spaced apertures 110 from the second side 114 of the implant 102. Then, the second end 122 of the length of suture 106 (i.e., tensioning limb 123) is passed through the other of the adjacent, spaced apertures 110 from the first side 112 of the implant 102 such that an intermediate portion 128A (FIG. 6) of the suture 106 extends between the adjacent, spaced apertures 110 on the first side 112 of the implant 102 (over the radiused saddle 112). Finally, the second end 122 of the length of suture 106 (i.e., tensioning limb 123) is passed through the adjustment mechanism 108. The tensioning limb 123 is slidable relative to the pierce hitch tail 121 (as the pierce hitch tail 121 does not slide).

Referring now to FIG. 6, there is shown a perspective view schematic representation of the anchor system 100, according to an embodiment. As shown in the depicted embodiment, the adjustment mechanism 108 is an eye splice. The eye splice 108 is formed in the length of suture 106 and the second end 122 of the length of suture 106 (i.e., tensioning limb 123) is passed through the eye splice 108, creating an adjustment loop 126 in the suture 106 between the first implant 102 and the eye splice 108. Thus, in the pre-deployment configuration, the intermediate portion 128A extends between the apertures 110 on the first side 112 of the first implant 102 and the intermediate portion 128B extends between the apertures 110 on the second side 114 of the second implant 104 with the eye splice 108 and the pierce hitch 116 therebetween. To adjust the adjustment loop 126 (i.e., change its diameter), the second end 122 of the suture 106 is pulled, moving the first implant 102 and second implant 104 closer together.

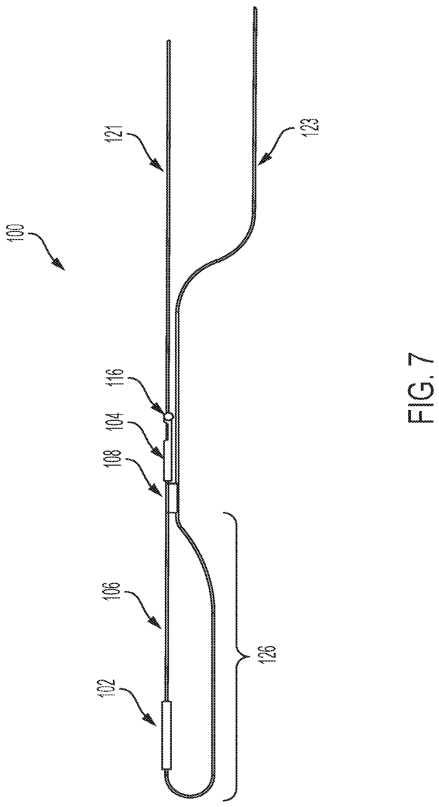

Turning now to FIGs. 7-10, there are shown various views schematic representations of the anchor system 100, according to alternative embodiments. FIG. 7 shows a side view of the anchor system 100 according to an alternative embodiment wherein the first and second implants 102, 104 are cannulated anchors (or any other tubular constructs). The cannulated anchors 102, 104 are composed of soft material, such as suture material. In the depicted embodiment, a length of suture 106 is folded in half, creating a sliding limb 123 and a non-sliding limb 121 (i.e., a tensioning limb 123 and a pierce hitch tail 121).

Still referring to FIG. 7, the length of suture 106 is passed through the first and second cannulated anchors 102, 104. A knot 116 is used to affix the second cannulated anchor 104 to the non-sliding limb 121. An eye splice 108 is formed in the non-sliding limb 121 between the first and second cannulated anchors 102, 104. The first cannulated anchor 102 is slidably connected to the suture 106 (the sliding limb 123). The sliding limb 123 is then passed through the eye splice 108, creating an adjustment loop 126, as shown in FIG. 7.

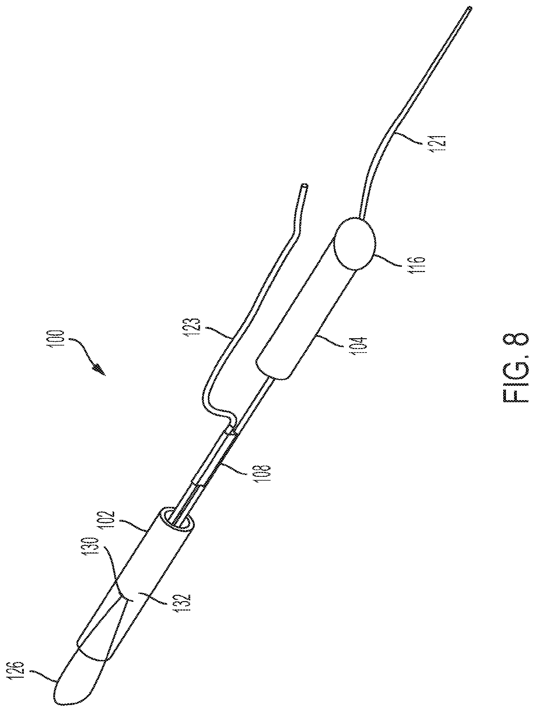

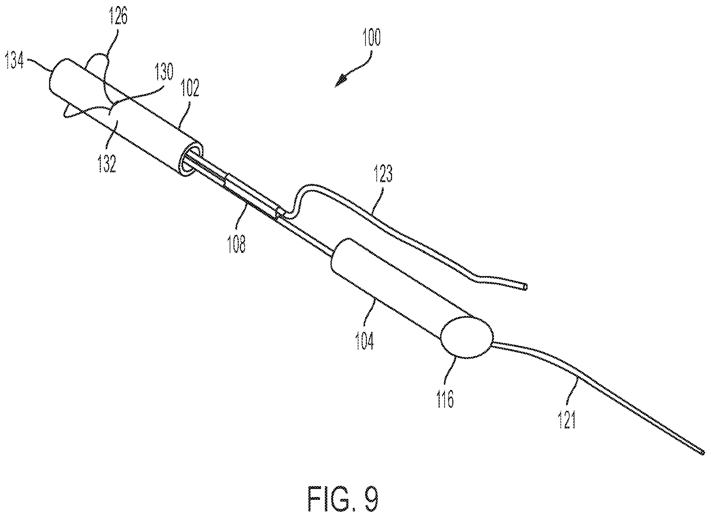

FIGs. 8 and 9 show perspective views schematic representations of the anchor system 100 of FIG. 7. In FIG. 8, the first cannulated anchor 102 is slidably attached to the suture 106 by passing the adjustment loop 126 through a slit 130 (or another type of segment) in the first cannulated anchor 102. Specifically, as shown in FIG. 8, the adjustable loop segment 102 is passed through the slit 130 in the first cannulated anchor 102 and exited out of a side 132 of the anchor 102. Then, the adjustable loop segment 126 is passed over a distal end 134 of the first cannulated anchor 102, thereby lassoing it, as shown in FIG. 9.

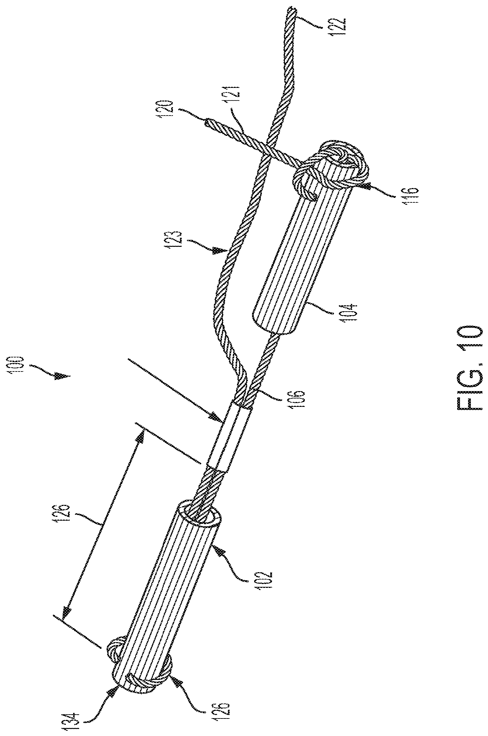

FIG. 10 shows a perspective view schematic representation of an anchor system 100, according to another alternative embodiment. The implants 102, 104 in FIG. 10 are also cannulated anchors (or any other tubular constructs). In the depicted embodiment, the cannulated anchors 102, 104 are composed of braided material. A length of suture 106 adjustably connects the first cannulated anchor 102 and the second cannulated anchor 104. In an embodiment, the length of suture 106 is a 2-0 suture, although other types of suture can be used. The length of suture 106 is folded in half so that both ends 120, 122 terminate on the same side and the other end forms a "U" shape (as also shown in FIG. 7). The second cannulated anchor 104 is secured to the suture 106 via a knot 116 in the length of suture 106. As with the previous embodiments of the anchor system 100, an eye splice 108 is formed in a locking limb 121 (also referred to herein as a non-sliding limb or pierce hitch tail) of the length of suture 106. The other limb, a tensioning limb 123, is passed through the eye splice 108, forming an adjustment loop 126. Similar to the anchor system 100 shown in FIGs. 8 and 9, the adjustment loop 126 extends around the distal end 134 of the first cannulated anchor 102.

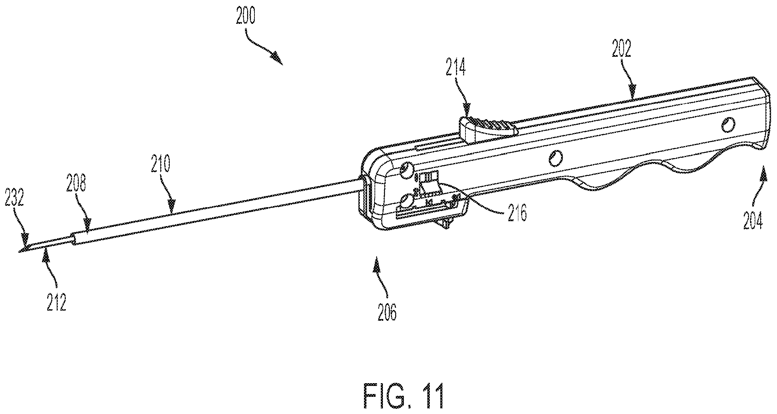

Referring now to FIGs. 11-17, there are shown various views schematic representations of a delivery device 200, according to an embodiment. The delivery device 200 is configured to store one or more anchor systems 100 therein for deployment. FIG. 11 shows a perspective view of the delivery device 200. The delivery device 200 comprises an elongated body 202 having a proximal end 204 and a distal end 206. The delivery device 200 comprises an adjustable introducer sleeve 210 and a stop (not shown) with a positive locking mechanism (not shown) extending from the distal end 206 of the elongated body 102. A needle 212 extends distally from within the introducer sleeve 210. The anchor system 100 (FIG. 6) is disposed completely within the needle 212 of the delivery device 200. A cannulated pusher rod 208 extends within a lumen 232 of the needle 212 of the delivery device 200. The purpose of the cannulated pusher rod 208 is to push the anchor system 100 from the delivery device 200, as described in detail below. The elongated body 202 additionally comprises an actuator 214 and a locking mechanism 216.

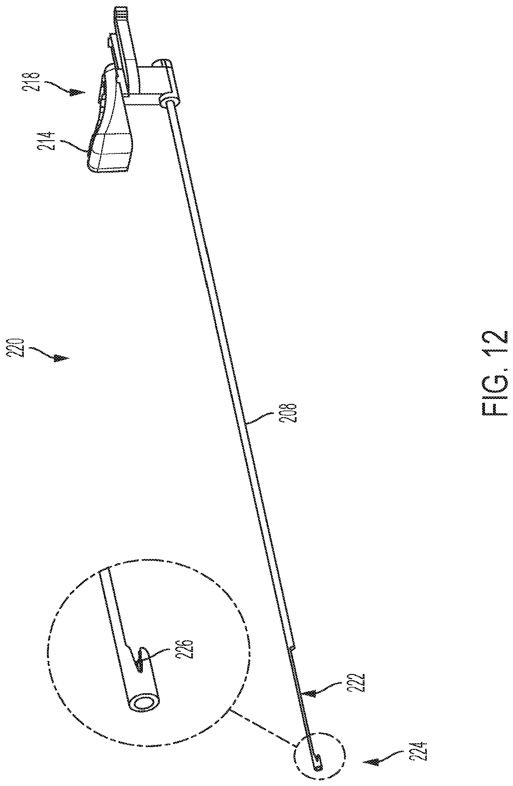

Turning now to FIG. 12, there is shown a perspective view schematic representation of a pusher assembly 220 of the delivery device 200, according to an embodiment. The pusher assembly 220 comprises a pusher body 218 with the cannulated pusher rod 208 extending distally therefrom. The pusher body 218 comprises the actuator 214. In the depicted embodiment, the actuator 214 is a thumb slide. The cannulated pusher rod 208 comprises a relieved area 222 between a distal end 224 of the pusher assembly 220 and the pusher body 218. The relieved area 222 is where a portion of the cannulated pusher rod 208 has been removed. The relieved area 222 allows for ease of rounding a corner or bend formed in the needle 212.

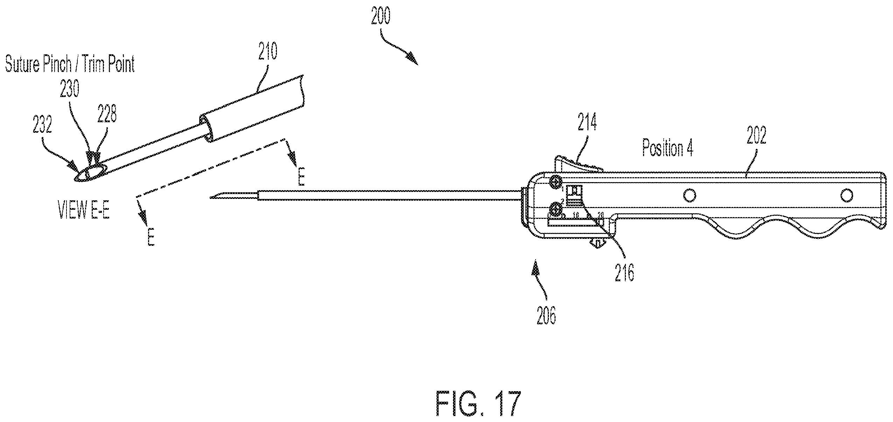

Still referring to FIG. 12, the cannulated pusher rod 208 also comprises a slit 226 at the distal end 224 of the pusher assembly 220. As shown, the slit 226 extends into the distal end 224 of the cannulated pusher rod 208 from the relieved area 222. This allows an end 120, 122 of suture 106 to be positioned within the slit 226. The cannulated pusher rod 208 can then be drawn proximally toward an opening 228 (FIG. 17) of the needle 212, creating a pinch point 230 between the cannulated pusher rod 208 and the lumen 232 of the needle 212 and severing an end 120, 122 of the suture 106.

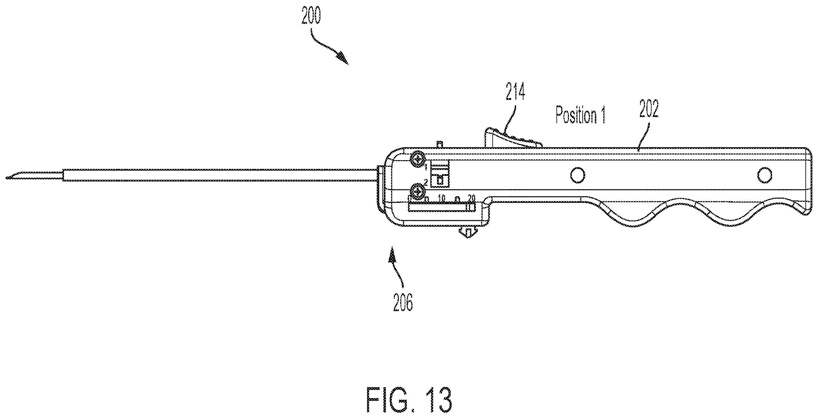

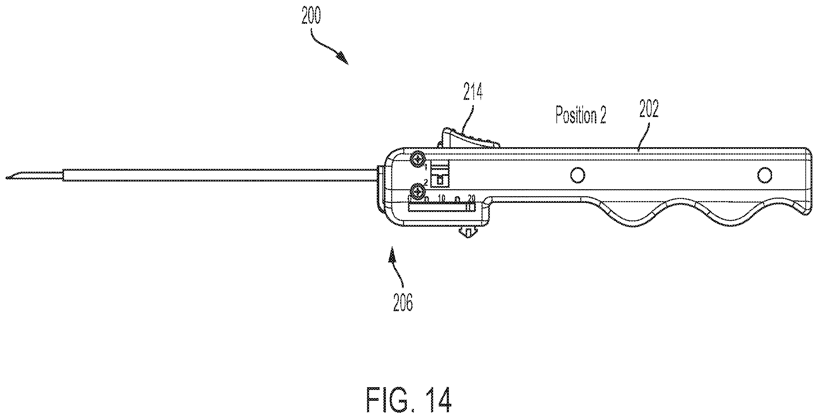

Turning now to FIGs. 13-17, there are shown side views schematic representations of the delivery device 200 in numerous configurations, according to an embodiment. In the first configuration, the actuator 214 is a first distance from the distal end 206 of the elongated body 202, as shown in FIG. 13. In the first configuration, delivery device 200 is prepared to deploy the anchor system 100. In the second configuration, the actuator 214 is a second distance from the distal end 206 of the elongated body 202, as shown in FIG. 14. In an embodiment, the first distance is greater than the second distance. When moving from the first configuration to the second configuration, the first implant 102 is deployed. Thus, by moving the actuator 214 (e.g., thumb slide) distally along the elongated body 202, the first implant 102 is deployed.

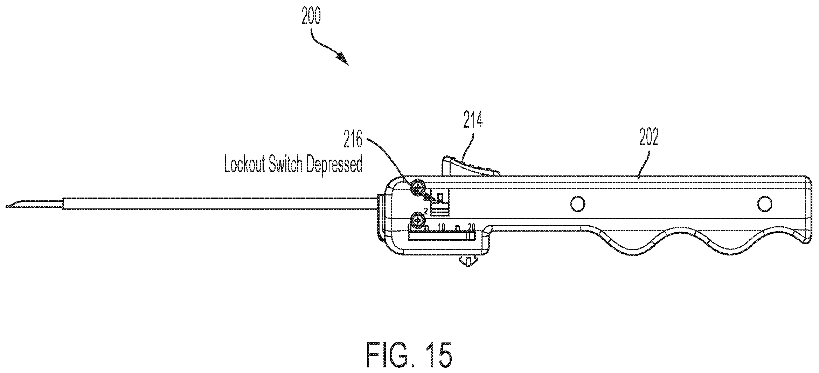

In both the first and second configurations, in FIGs. 13 and 14, the locking mechanism 216 is in the locked position. In the depicted embodiment, the locking mechanism 216 is a lockout switch. The lockout switch 216 is slidable or otherwise movable between a locked position and an unlocked position. In the locked position, as shown in FIGs. 13 and 14, the actuator 214 cannot advance distally from the second configuration in FIG. 14. The actuator 214 cannot advance distally along the elongated body 202 past the lockout switch 216. The lockout switch 216 can be depressed and moved to the unlocked position, as shown in FIG. 15

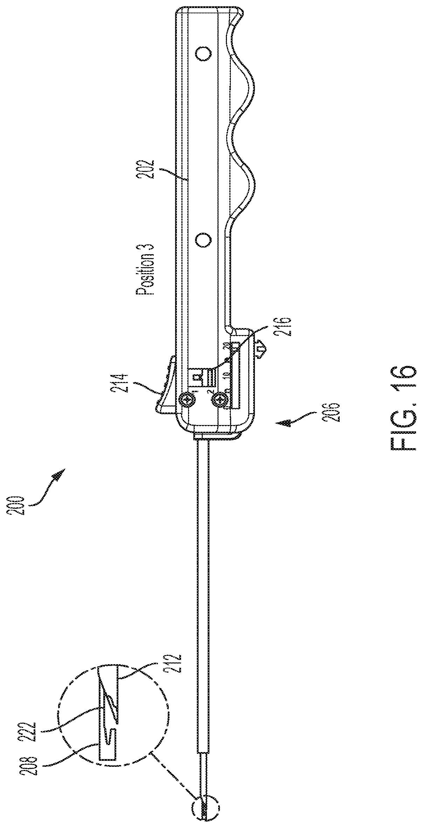

When the lockout switch 216 is in the unlocked position, as shown in FIG. 15, the actuator 214 can move distally to a third distance from the distal end 206 of the elongated body 202 to achieve the third configuration shown in FIG. 16. When moving from the second configuration to the third configuration, the pusher rod 208 is extended out of the needle 212, deploying the second implant 104. In the third configuration, the relieved area 222 is exposed between the pusher rod 208 and the needle 212, as shown.

In the third configuration, suture 106 can be moved into the relieved area 222 between the pusher rod 208 and the needle 212. With the end(s) 120, 122 of suture 106 extending through the relieved area 222, the delivery device 200 can be moved from the third configuration to a fourth configuration. To move the delivery device 200 from the third configuration to the fourth configuration, the actuator 214 is pulled in the proximal direction. When the delivery device 200 moves from the third configuration to the fourth configuration (FIG. 17), the cannulated pusher rod 208 is drawn back into the needle 212, allowing the end(s) 120, 122 of suture 106 to be cut against needle 212.

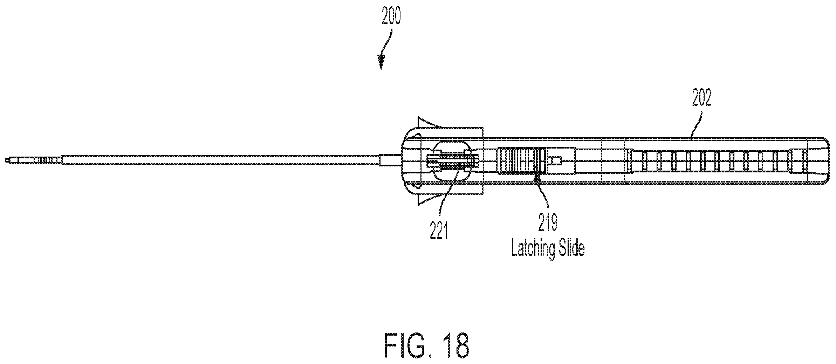

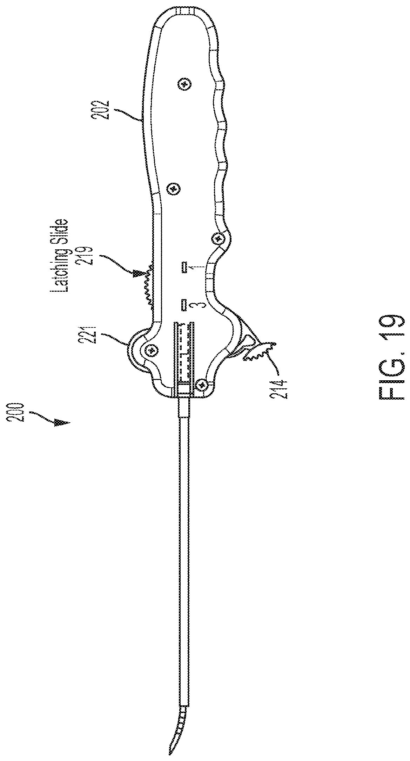

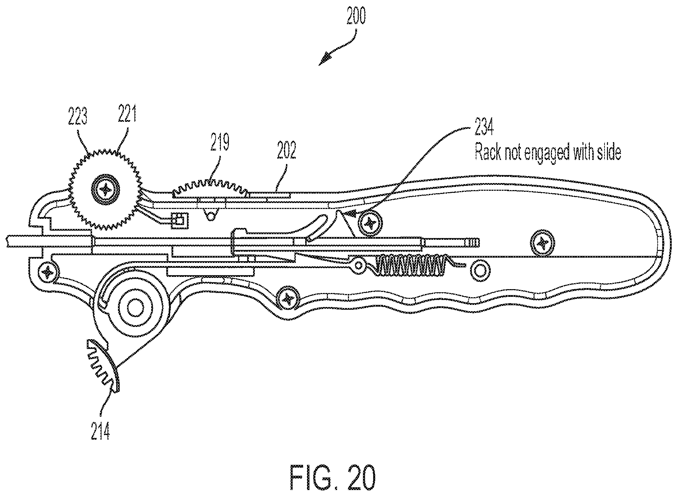

Referring now to FIGs. 18-22, there are shown various views schematic representations of a delivery device 200, according to an alternative embodiment. FIGs. 18 and 19 show an optional latching slide 219 and a tensioning wheel 221 of the elongated body 202 of the delivery device 200. The delivery device 200 also includes the actuator 214 on an opposing side of the elongated body 202 relative to the latching slide 219. As shown in FIG. 20, a rack 234 within the elongated body 202 connects to the cannulated pusher rod 208 and moves the cannulated pusher rod 208 in and out from the needle 212.

Still referring to FIG. 20, the actuator 214 comprises a ratchet mechanism for selectively advancing the cannulated pusher rod 208. When the actuator 214 is pulled in the proximal direction, the rack 234 is moved distally, driving the cannulated pusher rod 208 in the distal direction. The actuator 214 may have a spring return such that the actuator 214 returns back to its first (or starting) configuration when released. The tensioning wheel 221 extends partially through the elongated body 202 such that suture 106 extending within the needle 212 can wrap around the tensioning wheel 221. The tensioning wheel 221 provides traction on the suture 106 when the implants 102, 104 are deployed. In the depicted embodiment, the tensioning wheel 221 comprises teeth 223 for gripping the suture 106. The latching slide 219 is optional and can be used to selectively lock the tensioning wheel 221 against rotation.

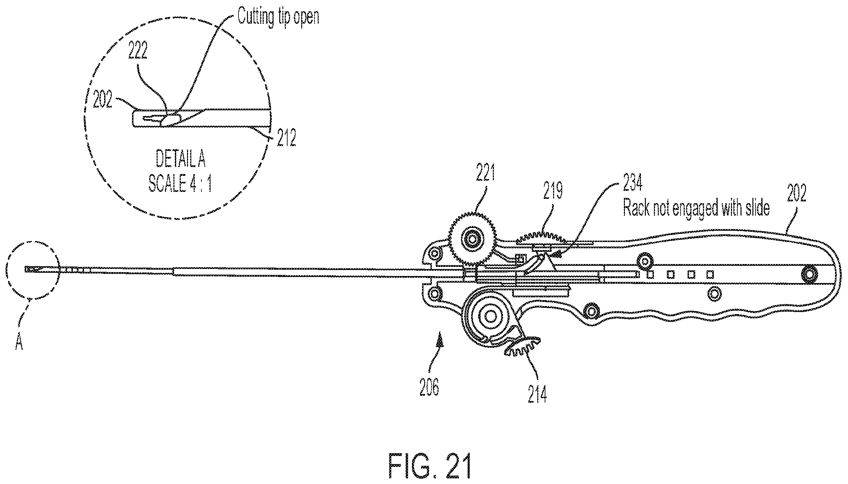

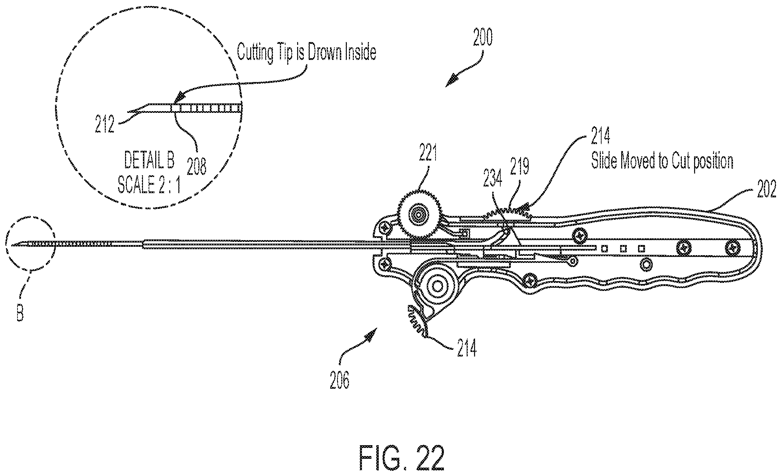

In a first configuration, as shown in FIGs. 19 and 22, the cannulated pusher rod 208 extends within the needle 212. The delivery device 200 can then be moved from the first configuration to a second configuration, shown in FIG. 21. To move the delivery device 200 from the first configuration to the second configuration, the actuator 214 is squeezed or pulled in the proximal direction. Moving the actuator 214 proximally, drives the rack 234 distally and as a result, pushes the cannulated pusher rod 208 distally out from the needle 212, expelling the first implant 102 from the needle 212. The actuator 214 can be released and then pulled proximally again when it is time to expel and deploy the second implant 104. The actuator 214 can also be maintained in the second configuration (FIG. 21) by holding the actuator 214 proximally, exposing the relieved area 222. With the relieved area 222 exposed, the ends 120, 122 of suture 106 can be extended into the relived area 222. When the actuator 214 is then released (FIG. 22), the end(s) 120, 122 of suture 106 are cut against the needle 212.

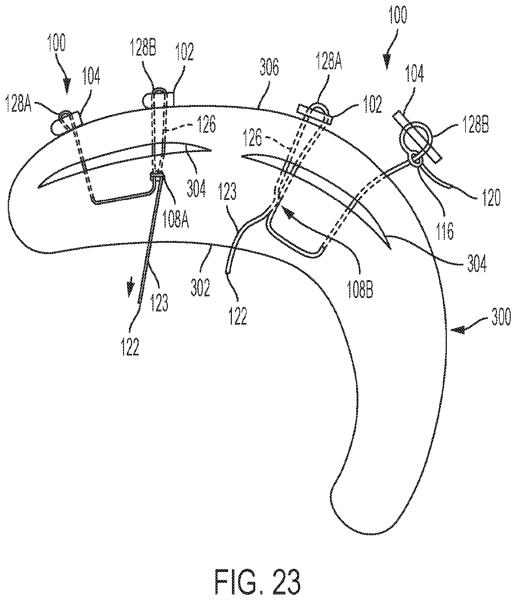

Referring now to FIGs. 23-25, there are shown various views schematic representations of the anchor system 100 in various stages between the pre-deployment configuration and deployed configuration, according to an alternative embodiment. As stated above, the anchor system 100 (FIG. 6) is disposed completely within the needle 212 of the delivery device 200. For example, the first implant 102 is loaded distally in the needle 212 relative to the second implant 104 with the pierce hitch tail 121 and the tensioning limb 123 extending proximally therefrom within the delivery device 200. (Note, it is contemplated that multiple anchor systems 100 can be loaded into the delivery device 200 with the first implant 102 of an additional anchor system 100 behind the second implant 104 of the primary anchor system 100).

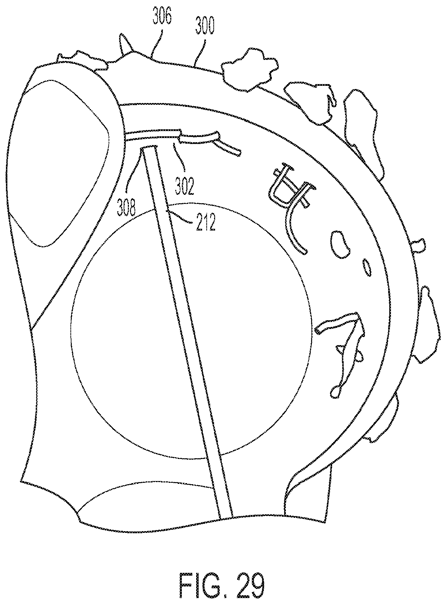

In an embodiment, the pierce hitch tail 121 is releasably connected to the delivery device 200 and functions as a tether used to control the delivery of the second implant 104 and apply traction to the first implant 102. To deploy the anchor system 100, the delivery device 200 is placed on a first side 302 of a tissue 300 or other object. As shown in FIG. 23, the tissue 300 comprises a tear 304 (or cut or other tissue injury). The delivery device 200, in the first configuration, is placed on the first side 302 of the tissue 300 and the tear 304. The needle 212 is advanced through the tissue 300 and the tear 304, at a first piercing location 308, and out of a second, opposing side 306 of the tissue 300 (FIG. 29).

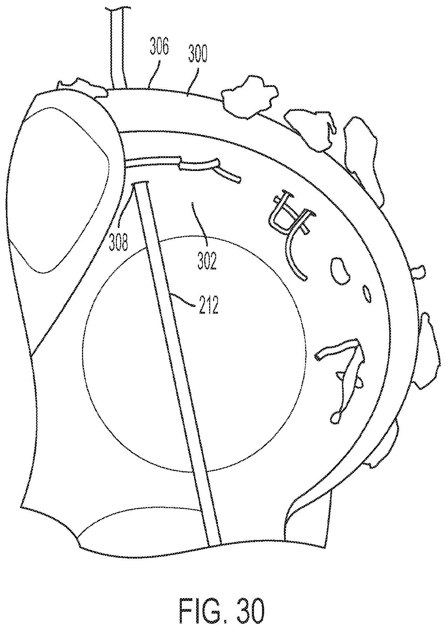

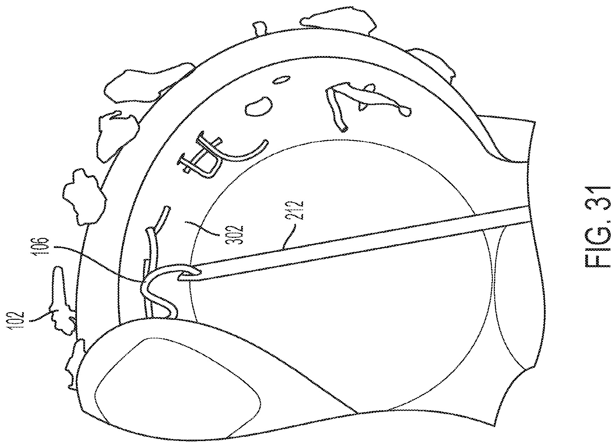

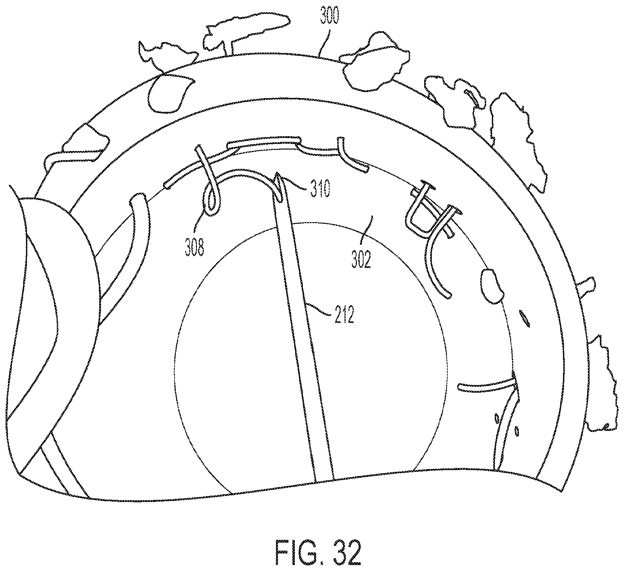

With the needle 212 on the second side 306 of the tissue 300, the actuator 214 is engaged, e.g., the thumb slide 214 is advanced in the distal direction along the elongated body 202, driving the cannulated pusher rod 208 distally to achieve the second configuration. In the second configuration, the cannulated pusher rod 208 pushes the first implant 102 from the delivery device 200 and it is deployed on the second side 306 of the tissue 300, as shown in FIG. 23. The needle 212 is then withdrawn from the first piercing location 308 on the first side 302 of the tissue 300 (FIGs. 30 and 31). In the embodiment of the delivery device 200 shown in FIGs. 18-22, the suture 106 can be tractioned by the tensioning wheel 221 as the needle 212 is withdrawn. The needle 212 is then moved to a second piercing location 310 (adjacent to the first piercing location 308) on the first side 302 of the tissue 300 and tear 304 (FIG. 32).

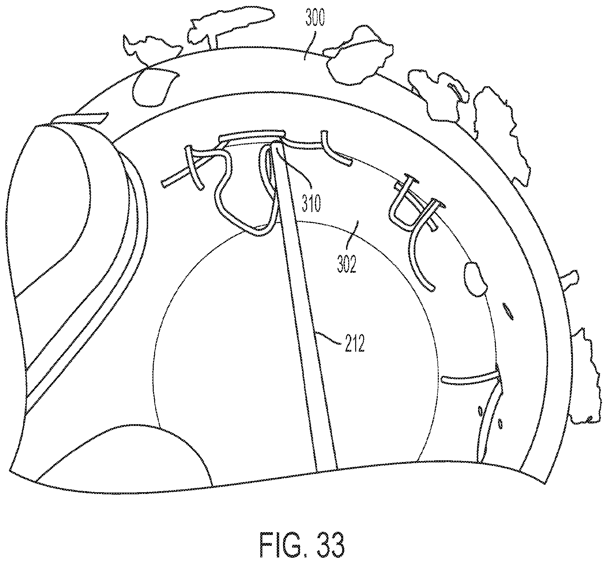

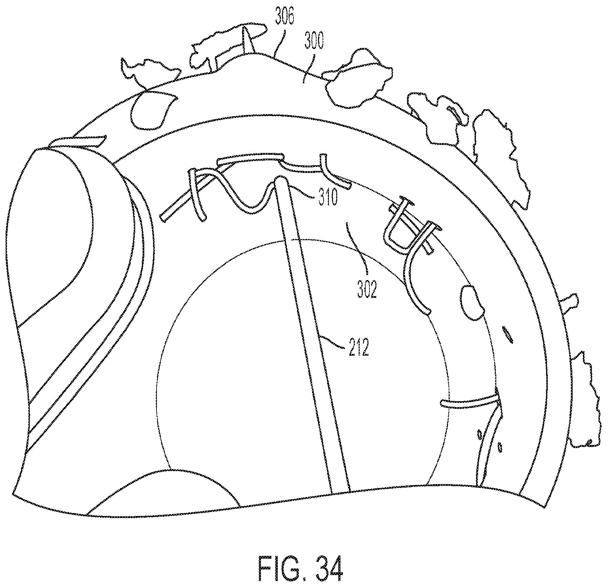

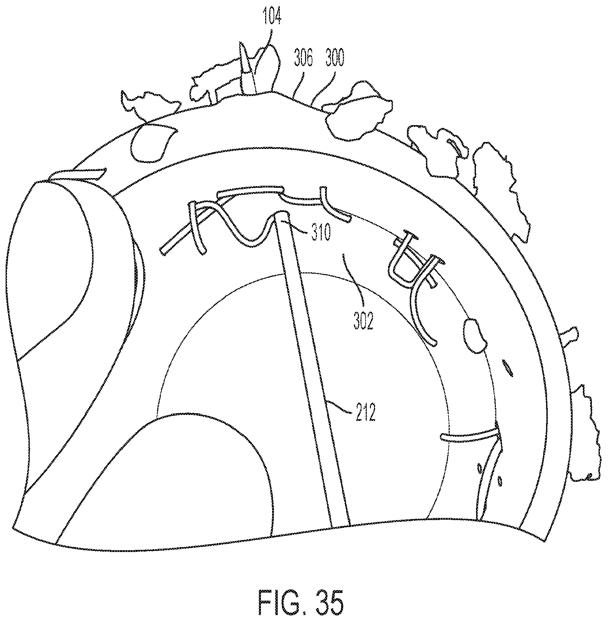

The needle 212 is then advanced through the first side 302, the tear 304, and the second side 306 of the tissue 300 at the second piercing location 310 (FIGs. 33-35). With the needle 212 on the second side 306 of the tissue 300, the delivery device 200 is moved to the third and fourth configurations. From the second configuration, the lockout switch 216 is depressed or otherwise engaged to allow for additional distal movement of the actuator 114. With the lockout switch 216 in the unlocked position, the actuator 114 is moved distally to the third configuration, driving the cannulated pusher rod 208 distally again. In the third configuration, the cannulated pusher rod 208 pushes the second implant 104 from the delivery device 200 and it is deployed, as also shown in FIG. 23.

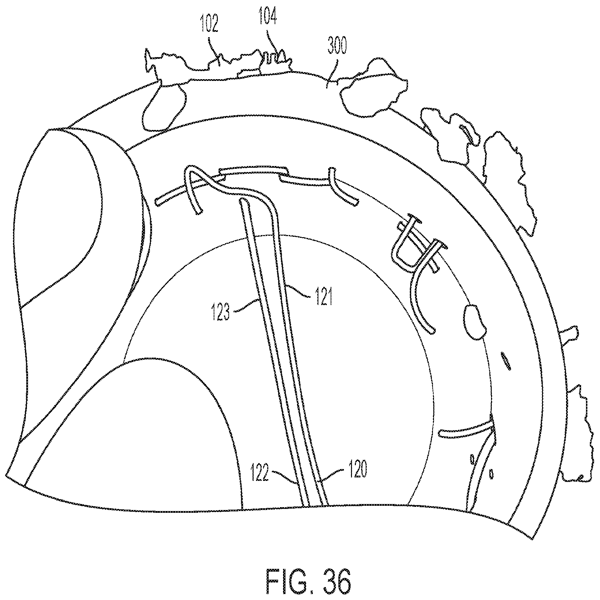

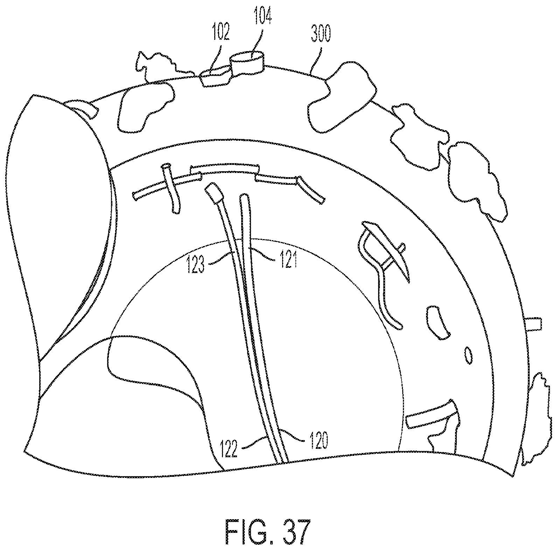

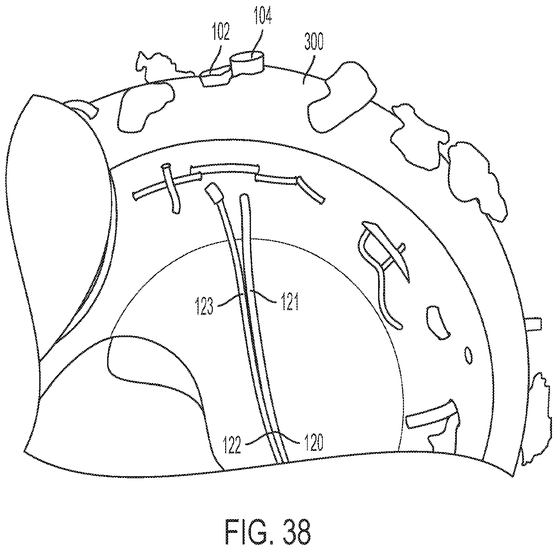

Any time after the second implant 104 is deployed, the pierce hitch tail 121 can be released (i.e., releasing the tether and traction), allowing for the delivery device 200 to be removed, i.e., the needle 212 is pulled back through the second piercing location 310 to the first side 302 of the tissue 300. With the anchor system 100 deployed, the anchor system 100 can be used to move the first side 302 of the tissue 300 and the second side 306 of the tissue 300 together in order to close the tear 304. To do this, the adjustable loop 126 is tightened (i.e., a diameter of the adjustable loop 126 decreases) or is otherwise collapsed by pulling/tensioning the second end 122 (or the tensioning limb 123) of the suture 106 (FIGs. 36-38). Tensioning the second end 122 pulls both implants 102, 104 toward the second side 306 of the tissue 300, decreasing the length of suture 106 between them. In FIG. 23, there are shown two anchor systems 100, each with a different adjustment mechanism 108. The anchor system 100 on the left comprises a sliding knot 108A and the anchor system 100 on the right comprises an eye splice 108B. The eye splice 108B (and the sliding knot 108A) locks the first implant 102 in relative position to the second implant 104. The pierce hitch 116 maintains the position of the second implant 104 along the suture 106.

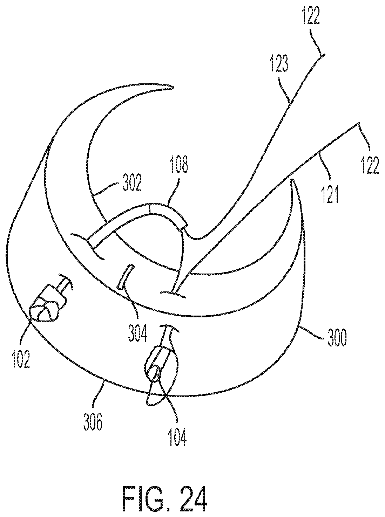

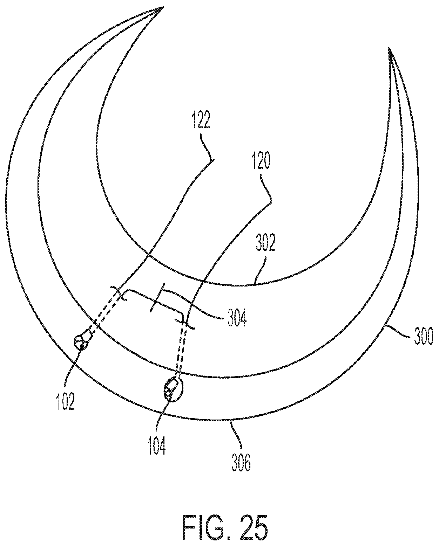

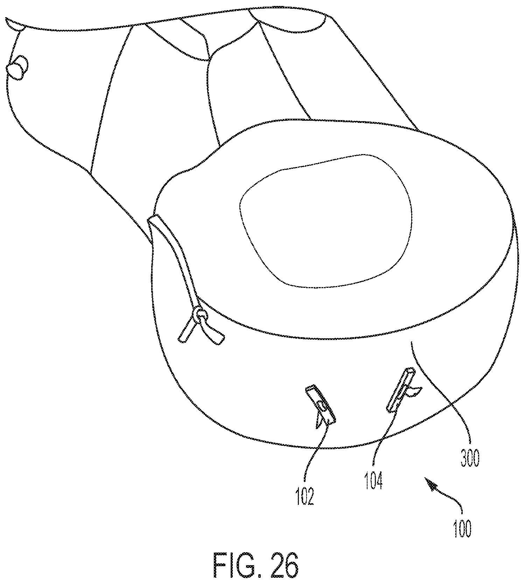

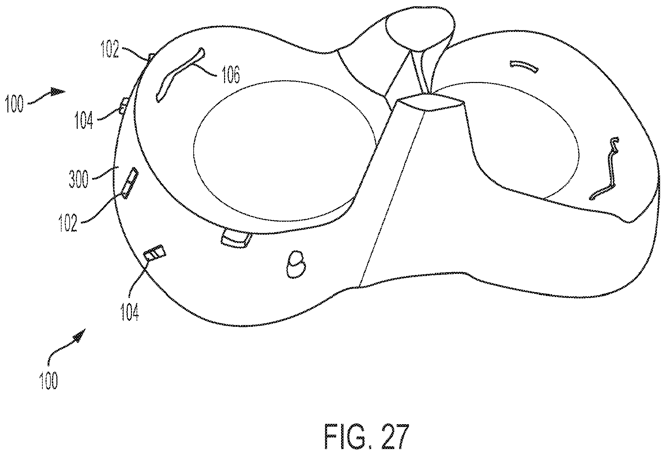

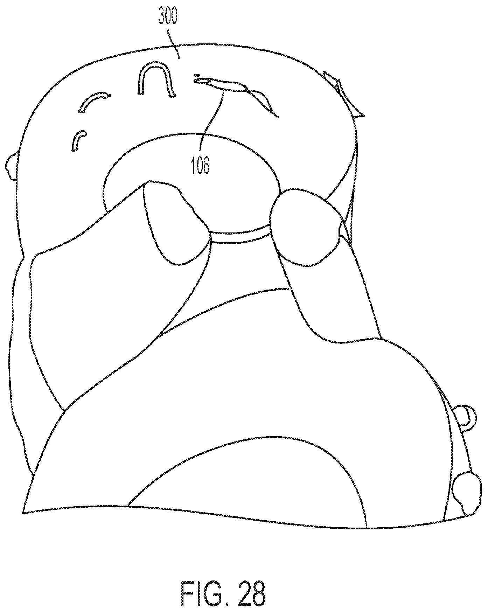

After the desired compression is achieved (between the first implant 102 and the second implant 104) and with the delivery device 200 still in the third configuration, the relieved area 122 is exposed between cannulated pusher rod 208 and the needle 212. The delivery device 200 can be rotated or otherwise maneuvered to receive the excess tensioning limb 123 in the relived area 122. The actuator 114 can then be engaged again, e.g., by sliding the thumb slide 114 back in the proximal direction (toward the proximal end 204 of the elongated body 202). Moving the actuator 214 proximally, pulls the cannulated pusher rod 208 proximally into the needle 212, allowing the excess tensioning limb 123 to be cut against the needle 212 and removed. The same process can be repeated (or occur simultaneously) with any excess pierce hitch tail 121 remaining. The resulting deployed configuration of the anchor system 100 is shown in FIGs. 26-28. FIGs. 24 and 25 show alternative embodiments wherein the implants 102, 104 are cannulated anchors.

All definitions, as defined and used herein, should be understood to control over dictionary definitions, definitions in documents and/or ordinary meanings of the defined terms.

While various embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the teachings is/are used. Those skilled in the art will recognize, or be able to ascertain using no more than routine experimentation, many equivalents to the specific embodiments described herein. It is, therefore, to be understood that the foregoing embodiments are presented by way of example only and that, within the scope of the appended claims and equivalents thereto, embodiments may be practiced otherwise than as specifically described and claimed. Embodiments of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the scope of the present disclosure.

The terminology used herein is for the purpose of describing particular embodiments only and is not intended to be limiting of the invention. As used herein, the singular forms "a", "an" and "the" are intended to include the plural forms as well, unless the context clearly indicates otherwise. It will be further understood that the terms "comprise" (and any form of comprise, such as "comprises" and "comprising"), "have" (and any form of have, such as, "has" and "having"), "include" (and any form of include, such as "includes" and "including"), and "contain" (any form of contain, such as "contains" and "containing") are open-ended linking verbs. As a result, a method or device that "comprises", "has", "includes" or "contains" one or more steps or elements. Likewise, a step of method or an element of a device that "comprises", "has", "includes" or "contains" one or more features possesses those one or more features, but is not limited to possessing only those one or more features. Furthermore, a device or structure that is configured in a certain way is configured in at least that way, but may also be configured in ways that are not listed.

The corresponding structures, materials, acts and equivalents of all means or step plus function elements in the claims below, if any, are intended to include any structure, material or act for performing the function in combination with other claimed elements as specifically claimed. The description of the present invention has been presented for purposes of illustration and description, but is not intended to be exhaustive or limited to the invention in the form disclosed. Many modifications and variations will be apparent to those of ordinary skill in the art without departing from the scope of the invention. The embodiment was chosen and described in order to best explain the principles of one or more aspects of the invention and the practical application, and to enable others of ordinary skill in the art to understand one or more aspects of the present invention for various embodiments with various modifications as are suited to the particular use contemplated.

## Claims

1. A delivery device for storing and deploying an anchor system comprising first and second implants (102; 104) interconnected by suture (106), comprising:
an elongated body (202) with a needle (212) extending distally therefrom;
a pusher assembly (220) within the elongated body, the pusher assembly comprising a cannulated pusher rod (208) extending distally from a pusher body (218) and an actuator (214) extending from the pusher body through the elongated body, the cannulated pusher rod being slidable within the needle;
a tensioning wheel (221) for providing traction on the suture while the first and second implants are deployed; and
a locking mechanism (216) on the elongated body, the locking mechanism movable between a locked position and unlocked position;
wherein the tensioning wheel and the actuator are arranged on opposite sides of the elongated body; and
wherein the actuator is moveable from a first configuration to a second configuration for deploying the first implant when the locking mechanism is in the locked position and the actuator is moveable from the second configuration to a third configuration for deploying the second implant when the locking mechanism is in the unlocked position.

2. The device of claim 1, further comprising a relieved area (222) between a distal end of the cannulated pusher rod (208) and the pusher body (218).

3. The device of claim 2, further comprising a slit (226) extending from the relieved area (222) into the pusher rod (208).

4. The device of claim 1, further comprising a pinch point (230) between the cannulated pusher rod (208) and a lumen (232) of the needle (212).

5. The device of claim 1, wherein the actuator (214) is a thumb slide.

## Patentansprüche

1. Übergabevorrichtung zum Aufnehmen und Einsetzen eines Ankersystems, das erste und zweite Implantate (102; 104) umfasst, die durch Nahtmaterial (106) miteinander verbunden sind, umfassend:
einen länglichen Körper (202) mit einer sich distal davon erstreckenden Nadel (212);
eine Schieberanordnung (220) innerhalb des länglichen Körpers, wobei die Schieberanordnung einen kanülierten Schiebestab (208), der sich distal von einem Schieberkörper (218) erstreckt, und ein Betätigungselement (214), das sich vom Schieberkörper durch den länglichen Körper erstreckt, umfasst, wobei der kanülierte Schiebestab innerhalb der Nadel gleiten kann;
ein Spannrad (221) zur Bereitstellung von Zugkraft auf das Nahtmaterial, während die ersten und zweiten Implantate eingesetzt werden; und
einen Verriegelungsmechanismus (216) am länglichen Körper, wobei der Verriegelungsmechanismus zwischen einer verriegelten Position und einer entriegelten Position bewegbar ist;
wobei das Spannrad und das Betätigungselement an entgegengesetzten Seiten des länglichen Körpers angeordnet sind; und
wobei das Betätigungselement von einer ersten Konfiguration zu einer zweiten Konfiguration zum Einsetzen des ersten Implantats bewegbar ist, wenn sich der Verriegelungsmechanismus in der verriegelten Position befindet, und das Betätigungselement von der zweiten Konfiguration zu einer dritten Konfiguration zum Einsetzen des zweiten Implantats bewegbar ist, wenn sich der Verriegelungsmechanismus in der entriegelten Position befindet.

2. Vorrichtung nach Anspruch 1, ferner umfassend einen entlasteten Bereich (222) zwischen einem distalen Ende des kanülierten Schiebestabs (208) und dem Schieberkörper (218).

3. Vorrichtung nach Anspruch 2, ferner umfassend einen Schlitz (226), der sich vom entlasteten Bereich (222) in den Schiebestab (208) erstreckt.

4. Vorrichtung nach Anspruch 1, ferner umfassend einen Zwickpunkt (230) zwischen dem kanülierten Schiebestab (208) und einem Lumen (232) der Nadel (212).

5. Vorrichtung nach Anspruch 1, wobei das Betätigungselement (214) ein Daumenschieber ist.

## Revendications

1. Dispositif de distribution pour stocker et déployer un système d'ancrage comprenant des premier et deuxième implants (102 ; 104) interconnectés par une suture (106), comprenant :
un corps allongé (202) avec une aiguille (212) s'étendant distalement depuis celui-ci ;
un ensemble (220) de poussoir à l'intérieur du corps allongé, l'ensemble de poussoir comprenant une tige canulée (208) de poussoir s'étendant distalement depuis un corps (218) de poussoir et un actionneur (214) s'étendant depuis le corps de poussoir à travers le corps allongé, la tige canulée de poussoir pouvant coulisser à l'intérieur de l'aiguille ;
une roulette (221) de mise en tension pour fournir une traction sur la suture tandis que les premier et deuxième implants sont déployés ; et
un mécanisme (216) de verrouillage sur le corps allongé, le mécanisme de verrouillage mobile entre une position verrouillée et une position déverrouillée ;
dans lequel la roulette de mise en tension et l'actionneur sont agencés sur des côtés opposés du corps allongé ; et
dans lequel l'actionneur est mobile d'une première configuration à une deuxième configuration pour déployer le premier implant lorsque le mécanisme de verrouillage est dans la position verrouillée et l'actionneur est mobile de la deuxième configuration à une troisième configuration pour déployer le deuxième implant lorsque le mécanisme de verrouillage est dans la position déverrouillée.

2. Dispositif selon la revendication 1, comprenant en outre une zone dégagée (222) entre une extrémité distale de la tige canulée (208) de poussoir et le corps (218) de poussoir.

3. Dispositif selon la revendication 2, comprenant en outre une fente (226) s'étendant depuis la zone dégagée (222) jusque dans la tige (208) de poussoir.

4. Dispositif selon la revendication 1, comprenant en outre un point (230) de pincement entre la tige canulée (208) de poussoir et une lumière (232) de l'aiguille (212).

5. Dispositif selon la revendication 1, dans lequel l'actionneur (214) est un coulisseau à pouce.
